# FASCICULE DE BREVET EUROPEEN

(11) **EP 1 263 525 B1**
(45) Date de publication et mention de la délivrance du brevet: **31.05.2006**
(21) Numéro de dépôt: 01911829.8
(22) Date de dépôt: 02.03.2001
(51) Int. Cl.: B01J 13/14, B01J 13/16

(54) **PROCEDE DE PREPARATION DE PARTICULES COLLOIDALES SOUS FORME DE NANOCAPSULES**
VERFAHREN ZUR HERSTELLUNG VON KOLLOIDALEN PARTIKELN, DIE IN FORM VON NANOKAPSELN SIND
METHOD FOR PREPARING COLLOIDAL PARTICLES IN THE FORM OF NANOCAPSULES

(30) Priorité: 10.03.2000 FR 0003133
(43) Date de publication de la demande: 11.12.2002
(73) Titulaire: UNIVERSITE CLAUDE BERNARD - LYON 1, F-69622 Villeurbanne Cédex (FR)
(72) Inventeur: MONTASSER, Imed, F-69100 Villeurbanne (FR); FESSI, Hatem, F-69003 Lyon (FR); BRIANCON, Stéphanie, F-01800 Saint Eloi (FR); LIETO, Joseph, F-69740 Genas (FR)
(74) Mandataire: Thibault, Jean-Marc
(86) Numéro de dépôt international: PCT/FR2001/000623
(87) Numéro de publication internationale: WO 2001/068235

(56) Documents cités:
- WO-A-94/13139
- WO-A-94/15590
- FR-A- 2 766 368
- US-A- 5 500 224
- PATENT ABSTRACTS OF JAPAN vol. 018, no. 506 (C-1252), 22 septembre 1994 (1994-09-22) & JP 06 170214 A (FUJI XEROX CO LTD), 21 juin 1994 (1994-06-21)
- PATENT ABSTRACTS OF JAPAN vol. 1995, no. 08, 29 septembre 1995 (1995-09-29) & JP 07 116500 A (FUJI XEROX CO LTD), 9 mai 1995 (1995-05-09)

## Description

L'invention a pour objet un nouveau procédé de préparation de particules colloïdales sous forme de nanocapsules par polycondensation interfaciale.

La vectorisation des principes actifs est une technique qui a pris un essor considérable et fait l'objet de nombreuses recherches ces dernières années, aboutissant à la conception de diverses formes de particules colloïdales associant une molécule active et un support. L'utilisation de vecteurs médicamenteux présente en effet l'avantage de pouvoir intervenir sur le processus de distribution du principe actif dans l'organisme et d'en augmenter l'efficacité. La molécule active incorporée dans un matériau support peut être orientée spécifiquement vers la cible à traiter où sa concentration est alors localement élevée. L'efficacité est ainsi accrue, tout en diminuant les doses nécessaires et par là aussi les risques d'effets secondaires par imprégnation d'autres organes.

L'association entre le matériau support et le principe actif peut se faire de diverses façons en fonction du type de particules et de l'effet recherché. La molécule active peut être dissoute, dispersée ou encapsulée dans la particule, ou alors elle peut être adsorbée ou fixée en surface de la particule. Dans le premier cas, la libération du principe actif se fera par dissolution du polymère constituant la particule ou sa membrane ou par diffusion à travers ce dernier. La nature et la structure du réseau de polymère et notamment sa porosité jouent alors un rôle fondamental. Dans le cas d'une liaison en surface, cette dernière doit être réversible pour libérer le principe au niveau de la cible à traiter. Il est également intéressant de modifier la nature du polymère utilisé afin de faire varier des caractéristiques telles que porosité, biodégradabilité, propriétés de transfert, ainsi que la biodisponibilité du principe actif.

Les méthodes de préparation de vecteurs colloïdaux sont diverses, chacune d'entre elles permettant l'utilisation de réactifs particuliers et conduisant à un type donné de particules.

Les méthodes faisant appel à une polymérisation en solution utilisent principalement deux types de monomères : les dérivés de l'acide acrylique et les cyanoacrylates d'alkyle. Les brevets BE-A-808 034, BE-A-839 748 décrivent la formation de particules submicroniques par polymérisation micellaire d'un dérivé de l'acide acrylique, par exemple le méthacrylate de méthyle ou de butyle, ou un mélange de plusieurs monomères afin de préparer un copolymère méthacrylique. La réaction de polymérisation s'effectue en phase aqueuse, en présence d'un initiateur chimique de radicaux libres, sous agitation, à une température voisine de 90°C. L'initiation peut également être faite par exemple par irradiation de rayons gamma. Les particules obtenues sont sphériques, de taille comprise entre 200 et 500 nm. Elles peuvent être utilisées comme vecteur de médicament par fixation sur leur surface de diverses molécules pharmacologiquement actives. Bien que leur formulation soit stable et reproductible, cette stabilité constitue un des inconvénients majeurs de ce type de particules. En effet, la plupart des polymères acryliques présentent une biodégradabilité très lente, voire nulle d'où la possibilité d'une accumulation du matériau dans les tissus.

La demande EP 007 895 décrit la formation de nanoparticules obtenues par polymérisation d'un cyanoacrylate d'alkyle et contenant une substance biologiquement active. Dans ce procédé, le monomère est ajouté à une phase aqueuse contenant un agent tensioactif, sous agitation importante. La polymérisation anionique, initiée par les ions hydroxyles de la phase aqueuse, a lieu à température ambiante. Le pH de la solution contrôle la vitesse de polymérisation et doit être faible (entre 2 et 3) pour optimiser la formation des particules. Le principe actif est généralement introduit pendant la polymérisation afin d'être incorporé aux particules, sauf s'il risque d'être dégradé par l'acidité du milieu. Il doit alors être adsorbé en surface après formation des particules. Les polymères à base de cyanoacrylate d'alkyle sont rapidement biodégradables, mais la toxicité des produits de dégradation, non négligeable, peut en limiter l'utilisation.

Les demandes FR-A-2 504 408 et FR-A-2 515 960 présentent une méthode de formation de nanocapsules à base de cyanoacrylate d'alkyle, dans laquelle le monomère cyanoacrylique est dissous dans un solvant miscible à l'eau contenant une huile. Cette solution est introduite dans une phase aqueuse sous agitation. La diffusion du solvant organique se produit simultanément à la polymérisation du monomère à l'interface huile/eau. Les nanocapsules ont un diamètre moyen compris entre 200 et 300 nm, avec une épaisseur de paroi très faible de quelques nanomètres selon ALKHOURI et Coll, Pharm. Acta Helv, 61, 274-281, 1986.

WO-A-9 415 590 décrit un procédé d'obtention de nanocapsules.

Le procédé selon l'invention permet l'obtention de nanocapsules à base de polymères nouvellement associés dans cette application, par une technique de polycondensation interfaciale de deux monomères. Dans un aspect avantageux, lesdites nanocapsules ont un diamètre moyen inférieur à 600 nm, notamment compris entre 50 et 600 nm.

L'invention a donc pour objet un procédé de préparation de systèmes colloïdaux dispersibles, sous forme de nanocapsules dont la paroi est constituée par un polymère obtenu par polycondensation de deux monomères α et β et dont le coeur est constitué par une substance B, caractérisé en ce que :
(1) on prépare une première phase liquide constituée par une solution de monomère α dans un solvant ou un mélange de solvants, et contenant au moins un agent tensioactif ainsi que la substance B en solution ou en suspension,
(2) on prépare une seconde phase liquide constituée par un non-solvant ou un mélange de non-solvants du monomère α et de la substance B, contenant le monomère β et au moins un agent tensioactif ; le solvant ou le mélange de solvants de la première phase étant miscible en toutes proportions au non-solvant ou au mélange de non-solvants de la seconde phase, et la concentration du monomère β étant en excès d'au moins 5 fois en nombre de moles par rapport à la concentration du monomère α,
(3) on ajoute, sous agitation modérée, la première phase à la seconde, de manière à obtenir une suspension colloïdale de nanocapsules, l'agitation étant maintenue jusqu'à polymérisation complète des monomères α et β,
(4) si on le désire, on élimine tout ou partie du solvant ou du mélange de solvants et de non-solvant ou du mélange de non-solvants de manière à obtenir une suspension colloïdale de concentration voulue en nanocapsules.

A l'étape (4), on peut également, si on le désire, obtenir une poudre de nanocapsules en mettant en oeuvre une technique de dessiccation (nébulisation, lyophilisation), après addition de substances stabilisantes telles que des sucres, lactose, glucose.

Contrairement aux procédés décrits précédemment, les 2 monomères sont mis en oeuvre dès le départ dans les 2 phases, et non successivement après l'obtention d'une nanoémulsion stable dans la première phase.

Le solvant ou le mélange de solvants de la première phase étant miscible en toutes proportions au non-solvant ou au mélange de non-solvants de la seconde phase, sa diffusion vers la seconde phase lors de l'injection entraîne la formation instantanée de gouttelettes huileuses de diamètre moyen inférieur à 300 nm. Simultanément, le monomère β diffuse vers la première phase et la réaction de polycondensation a lieu à l'interface huile/eau formant la membrane des nanocapsules.

La formation des gouttes et le début de la polymérisation ont lieu simultanément. La polymérisation n'est pas libre, elle a effectivement lieu à l'interface huile/eau et les particules formées sont de type capsules. L'originalité du procédé selon l'invention réside dans le fait que les deux monomères présents initialement dans chacune des phases réagissent à l'interface des gouttelettes dès la formation de ces dernières, et ce malgré la faible taille de la dispersion. Il n'est donc pas nécessaire de mettre en oeuvre un processus en deux phases successives comprenant la création de l'émulsion dans un premier temps, suivie de l'addition du deuxième monomère pour débuter la polymérisation.

Par rapport à une polymérisation en émulsion, qui demande souvent une méthodologie longue et difficile à mettre en oeuvre, le procédé selon l'invention présente donc l'avantage d'une grande simplicité dans la mesure où il ne nécessite pas la présence d'un initiateur de polymérisation ni de dispositif particulier pour créer l'émulsion.

La formation des particules est instantanée. Cependant, la réaction de polymérisation a lieu avec une cinétique dépendant de la nature chimique et de la concentration des deux monomères dans les 2 phases.

Le solvant ou le mélange de solvants de la première phase est avantageusement un solvant organique ou un mélange de solvants organiques, de sorte que la première phase constituera la phase organique, et le non-solvant ou le mélange de non-solvants de la seconde phase constituera la phase aqueuse.

Selon un autre aspect du procédé selon l'invention, on peut également utiliser deux phases organiques ou deux phases aqueuses dans la mesure où sont remplies les conditions de solubilité du monomère α dans le solvant ou le mélange de solvants de la première phase, d'insolubilité du monomère. α dans le non-solvant ou le mélange de non-solvants de la seconde phase, de miscibilité du solvant ou du mélange de solvants de la première phase et du non-solvant ou du mélange de non-solvants de la seconde phase, ainsi que de leur non-réactivité avec les monomères.

Dans la mesure où il n'est pas réactif avec le monomère α, le solvant peut être par exemple un solvant organique, de préférence volatil, choisi parmi une cétone inférieure (acétone, méthyléthylcétone, etc...), un hydrocarbure léger ou un mélange d'hydrocarbures légers (hexane, etc...), un hydrocarbure léger chloré (chloroforme, chlorure de méthylène), d'autres solvants usuels (acétonitrile, dioxane, tétrahydrofurane, etc...), et leurs mélanges.

Dans la mesure où les conditions de solubilité, d'insolubilité, de miscibilité et de non réactivité avec les monomères sont remplies, le solvant ou le mélange de solvants de la première phase peut constituer de 10 à 90% du mélange final, de préférence de 20 à 60% du mélange final, de préférence encore de 25 à 50%.

Le non- solvant ou le mélange de non-solvants, dans la mesure où il n'est pas réactif avec le monomère β et où il est miscible en toutes proportions au solvant ou au mélange de solvants de la première phase, peut être par exemple de l'eau ou une solution aqueuse ou tout autre solvant ou liquide organique remplissant les conditions précitées.

Avantageusement, la concentration du monomère α dans le solvant ou le mélange de solvants de la première phase est comprise entre 0,01 et 20% en poids de préférence entre 0,1 et 10 % et plus préférentiellement entre 0,2 et 5 %. La concentration du monomère β dans le non-solvant ou le mélange de non-solvants de la seconde phase peut également être comprise entre 0,05 et 50% en poids de préférence entre 0,5 et 40 % et plus préférentiellement entre 1 et 25 %.

Dans un aspect préféré, le monomère α est choisi parmi les dichlorures d'acides et les diisocyanates. Des monomères α particulièrement préférés sont le chlorure de téréphtaloyle, le chlorure de sébacoyle, le toluylène-2,4-diisocyanate et l'hexaméthylène diisocyanate.

Selon un autre aspect préféré de l'invention, le monomère β est une diamine, par exemple la diéthylène triamine, la diéthylène diamine ou l'hexaméthylène diamine, ou un dérivé de glycol.

Dans un aspect avantageux, la concentration du monomère β est en excès par rapport à celle du monomère α, de préférence en excès d'au moins 5 fois en nombre de moles.

En effet, de manière surprenante, on a trouvé que ces conditions conferent une grande stabilité aux nanocapsules et permettent, en augmentant la concentration en monomère α, d'augmenter l'épaisseur de la membrane des nanocapsules à volonté, ce qui conduit à une augmentation du diamètre moyen des nanocapsules. Comme le montre le tableau ci-dessous et sachant que toutes les autres proportions étant gardées, cette augmentation du diamètre moyen ne peut être expliquée que par une augmentation de l'épaisseur de la membrane des nanocapsules.

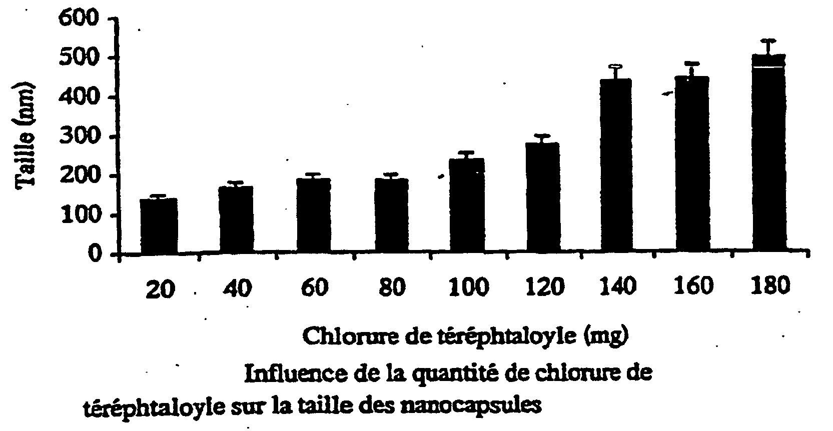

Une hypothèse à cet égard est que la polymérisation continue à se produire au niveau de la membrane des nanocapsules, alors que selon les procédés décrits dans l'art antérieur, la polymérisation s'arrête après la formation d'une première membrane polymérique fine.

Le procédé selon l'invention permet donc avantageusement, selon les monomères mis en oeuvre et l'épaisseur de la membrane polymérique, d'obtenir des nanocapsules ayant une biodégradabilité contrôlée, voire si on le souhaite, des nanocapsules ayant une membrane non biodégradable et insoluble, pouvant constituer un réservoir de principe actif dont la libération s'effectue uniquement par diffusion à travers la membrane polymérique.

La substance B peut être n'importe quelle substance soluble ou dispersible dans le solvant ou le mélange de solvants choisi. En particulier, la substance B peut être une huile végétale ou minérale, ou toute substance huileuse, par exemple l'huile d'olive, le benzoate de benzyle, le myristate d'isopropyle, des glycérides d'acide gras ou l'huile d'amande douce.

La substance B peut également être une substance biologiquement active, par exemple une molécule utilisable comme principe actif de médicament ou comme précurseur d'un principe actif de médicament, ou encore un produit de contraste ou un réactif biologique.

La substance B peut également être un pigment, une encre, un lubrifiant ou un agent de traitement de surface.

On peut également utiliser en tant que substance B un mélange des substances ci-dessus, par exemple une huile contenant une ou plusieurs de ces substances en solution ou en suspension.

Les agents tensioactifs utilisés peuvent être des agents tensioactifs naturels, ou des agents tensioactifs synthétiques ioniques, non ioniques ou amphotères.

Dans chacune des phases, l'agent tensioactif ou le mélange d'agents tensioactifs est présent à raison de 0,01 à 10% en poids, de préférence 0,1 à 1% en poids.

En tant qu'agent tensioactif ionique, on utilisera par exemple le laurylsulfate de sodium.

En tant qu'agent tensioactif non ionique, et selon la phase dans laquelle ils seront mis en oeuvre, on utilisera de préférence des agents tensioactifs dont la balance hydrophile/lipophile (de l'anglais «HLB» : hydrophilic/lipophilic balance) est élevée tels que, par exemple, les dérivés de sorbitanne polyoxyéthylénés (de type Tween® ), des copolymères d'oxyde d'éthylène et de propylène (de type Pluronic®) ou des éthers d'alcools gras et de polyoxyéthylèneglycol ou au contraire des agents tensioactifs dont la balance hydrophilellipophile est basse, tels que les dérivés de sorbitanne (de type Span®).

En tant qu'agent tensioactif amphotère, on utilisera par exemple la lécithine d'oeuf ou de soja ou ses dérivés purifiés.

Selon un aspect préféré du procédé, la première phase est une phase organique et on utilise comme agent tensioactif un ou plusieurs agent(s) tensioactif(s) amphotères et/ou non ioniques, de préférence, ceux dont la balance hydrophile/lipophile est basse parmi ceux mentionnés ci-dessus.

Avantageusement, la seconde phase est une phase aqueuse dans laquelle on utilise comme agent tensioactif un ou plusieurs agent(s) tensioactif(s) ioniques et/ou non ioniques, de préférence ceux dont la balance hydrophile/lipophile est élevée parmi ceux mentionnés ci-dessus.

La réaction a lieu à température ambiante, sous agitation modérée. La durée de polymérisation est variable et dépend de la composition de chacune des phases.

L'agitation n'est pas indispensable pour la formation des nanocapsules mais permet d'homogénéiser la préparation, notamment lorsqu'on utilise de grands voiumes.

Lorsque la polymérisation est complète, le solvant ou le mélange de solvants ainsi que le non solvant ou le mélange de non-solvants du mélange final peuvent être au moins pour partie, éliminés par évaporation sous pression réduite ou par une méthode de dessiccation appropriée ou encore par ultrafiltration tangentielle, cette technique permettant également d'éliminer les éventuels monomères résiduels.

Les nanocapsules obtenues ont un diamètre moyen compris entre 50 et 600 nm, leur population étant monodispersée. Les nanocapsules peuvent être conservées en milieu aqueux.

Le procédé selon l'invention peut être adapté à divers couples de monomères pour former différents types de polymères selon l'application envisagée, par exemple polyamide, polyurée, polyuréthane, polyester, polycarbonate, polysulfonate, polysulfonamide, etc.

Le large choix des couples des monomères permet d'avoir des nanocapsules de biodégradabilité contrôlée (selon l'épaisseur de la membrane et la nature du couple choisi), ce que ne peut offrir l'art antérieur.

Selon les monomères, et contrairement à l'art antérieur, on peut obtenir une membrane non biodégradable et insoluble et on obtient ainsi un réservoir du produit actif, dont la libération s'effectue uniquement par diffusion à travers la membrane polymérique.

Le choix de différents types de polymères permet d'utiliser les particules formées pour des applications très variées dans de nombreux domaines industriels et notamment en médecine humaine et vétérinaire, cosmétique, chimie, agrochimie, etc.

L'invention est illustrée de manière non limitative par les exemples ci-après :

### EXEMPLE 1 : Préparation de nanocapsules de polyamide

On prépare une solution organique en dissolvant, dans 20 ml d'acétone, 100 mg de chlorure de téréphtaloyle, 200 mg d'huile Miglyol® 812 (huile neutre formée d'un mélange de triglycérides d'acide gras en C₈-C₁₀) et 40 mg de lécithine (Lipoïd® S75).

On prépare une phase aqueuse en dissolvant, dans 40 ml d'eau, 500 mg de diéthylènetriamine et 60 mg de Pluronic® F68 (polymère mixte d'oxyde d'éthylène et de propylène glycol). On injecte la phase organique dans la phase aqueuse, les nanocapsules se forment instantanément. On maintient une agitation magnétique modérée (500 r.p.m.) jusqu'à la fin de la polycondensation. Enfin, on élimine le solvant organique et une partie de l'eau par évaporation. Le diamètre moyen des nanocapsules mesuré à l'aide d'un granulomètre à rayon laser (COULTER® LS 230) est de 300 nm.

### EXEMPLE 2 :

On procède comme dans l'exemple 1, mais en remplaçant la diéthylènetriamine par la diéthylènediamine. On obtient des nanocapsules ayant un diamètre moyen de 285 nm.

### EXEMPLE 3 :

On procède comme dans l'exemple 1, mais en remplaçant les 500 mg de diéthylènetriamine par 500 mg d'hexaméthylènediamine. On obtient des nanocapsules ayant un diamètre moyen de 500 nm.

### EXEMPLE 4 :

On procède comme dans l'exemple 1, mais en remplaçant les 100 mg de chlorure de téréphtaloyle par 100 mg de chlorure de sébacoyle. On obtient des nanocapsules ayant un diamètre moyen de 300 nm.

### EXEMPLE 5 :

On procède comme dans l'exemple 1, mais en augmentant la quantité de Miglyol® 812 à 300mg. On obtient des nanocapsules ayant un diamètre moyen de 415nm.

### EXEMPLE 6 :

On procède comme dans l'exemple 1, mais en augmentant la quantité de chlorure de téréphtaloyle à 140 mg. On obtient des nanocapsules ayant un diamètre moyen de 440 nm.

### EXEMPLE 7 :

On procède comme dans l'exemple 1, mais en ajoutant dans la phase acétonique 20 mg de Span® 80. On obtient des nanocapsules ayant un diamètre moyen de 280 nm.

### EXEMPLE 8 :

On procède comme dans l'exemple 1, mais en ajoutant dans la phase aqueuse, 30 mg de Tween® 80. On obtient des nanocapsules ayant un diamètre moyen de 290 nm.

### EXEMPLE 9 : Préparation de nanocapsules de polyurée

On procède comme dans l'exemple 1, mais en remplaçant les 100 mg de chlorure de téréphtaloyle par 100 mg de toluylène-2,4-diisocyanate. Les nanocapsules obtenues ont un diamètre moyen de 120 nm.

### EXEMPLE 10 :

On procède comme dans l'exemple 1, mais en remplaçant les 100 mg de chlorure de téréphtaloyle par 100 mg d'hexaméthylène diisocyanate. Les nanocapsules obtenues ont un diamètre moyen de 118 nm.

### EXEMPLE 11 : Préparation de nanocapsules de polyamide contenant de la lidocaïne

On prépare une solution organique en dissolvant, dans 20 ml d'acétone, 100 mg de chlorure de téréphtaloyle, 40 mg de lidocaïne dissous dans 200 mg d'huile (Miglyol® 812), et 40 mg de lécithine (Lipoïd® S75).

On prépare une phase aqueuse en dissolvant, dans 40 ml d'eau 500 mg de diéthylènetriamine et 60 mg de Pluronic® F68.

On injecte la phase organique dans la phase aqueuse, les nanocapsules se forment instantanément.

On maintient l'agitation jusqu'à la fin de la polycondensation. Enfin, on élimine le solvant organique et une partie de l'eau par évaporation. Les nanocapsules obtenues ont un diamètre moyen de 318 nm.

Après un repos prolongé de plusieurs jours, l'aspect blanc à reflets bleutés de la suspension demeure inchangé et on n'observe, en particulier, ni rupture ni variation considérable de la taille des nanocapsules.

### EXEMPLE 12 : Préparation de nanocapsules de polyamide contenant de la progestérone

On procède comme dans l'exemple 9, mais en ajoutant 5 mg de progestérone dans la phase organique. Les nanocapsules obtenues ont un diamètre moyen de 148 nm. La suspension garde son aspect après plusieurs jours de stockage.

## Revendications

1. Procédé de préparation de systèmes colloïdaux dispersibles, sous forme de nanocapsules dont la paroi est constituée par un polymère obtenu par polycondensation de deux monomères α et β et dont le coeur est constitué par une substance B, **caractérisé en ce que** :
(1) on prépare une première phase liquide constituée par une solution de monomère α dans un solvant ou un mélange de solvants, et contenant au moins un agent tensioactif ainsi que la substance B en solution ou en suspension,
(2) on prépare une seconde phase liquide constituée par un non-solvant ou un mélange de non-solvants du monomère α et de la substance B,contenant le monomère β et au moins un agent tensioactif ; le solvant ou le mélange de solvants de la première phase étant miscible en toutes proportions au non-solvant ou au mélange de non-solvants de la seconde phase et la concentration du monomère β étant en excès d'au moins 5 fois en nombre de moles par rapport à la concentration du monomère α,
(3) on ajoute, sous agitation modérée, la première phase à la seconde, de manière à obtenir une suspension colloïdale de nanocapsules, l'agitation étant maintenue jusqu'à polymérisation complète des monomères α et β,
(4) si on le désire, on élimine tout ou partie du solvant ou du mélange de solvants et de non-solvant ou du mélange de non-solvants de manière à obtenir une suspension colloïdale de concentration voulue en nanocapsules.

2. Procédé selon la revendication 1, **caractérisé en ce que** le solvant ou le mélange de solvants de la première phase est un solvant organique ou un mélange de solvants organiques et le non-solvant ou le mélange de non-solvants de la seconde phase constituent une phase aqueuse.

3. Procédé selon l'une des revendications 1 ou 2, **caractérisé en ce que** la concentration du monomère α dans le solvant ou le mélange de solvants de la première phase est comprise entre 0,01 et 20% en poids, de préférence entre 0,1 et 10 % et plus préférentiellement entre 0,2 et 5 %.

4. Procédé selon l'une quelconque des revendications 1 à 3, **caractérisé en ce que** la concentration du monomère β dans le non-solvant ou le mélange de non-solvants de la seconde phase est comprise entre 0,05 et 50 % en poids, de préférence entre 0,5 et 40 % et plus préférentiellement entre 1 et 25 %.

5. Procédé selon l'une quelconque des revendications 1 à 4, **caractérisé en ce que** le monomère α est choisi parmi les dichlorures d'acides et les diisocyanates.

6. Procédé selon l'une quelconque des revendications 1 à 5, **caractérisé en ce que** le monomère β est une diamine ou un dérivé de glycol.

7. Procédé selon l'une quelconque des revendications 1 à 6, **caractérisé en ce que** la substance B est une huile végétale ou minérale ou toute substance huileuse.

8. Procédé selon l'une quelconque des revendications 1 à 6, **caractérisé en ce que** la substance B est une substance biologiquement active.

9. Procédé selon l'une quelconque des revendications 1 à 6, **caractérisé en ce que** la substance B est une huile contenant une ou plusieurs substance(s) biologiquement active(s) en solution ou en suspension.

10. Procédé selon l'une quelconque des revendications 1 à 9, **caractérisé en ce que** l'agent tensioactif ou le mélange d'agents tensioactifs est présent dans chaque phase à raison de 0,01 à 10% en poids, de préférence 0,1 à 1 % en poids.

11. Procédé selon l'une quelconque des revendications 1 à 10, **caractérisé en ce que** la première phase est une phase organique et on utilise comme agent tensioactif au moins un agent tensioactif amphotère et/ou non ionique, de préférence ayant une balance hydrophile/lipophile basse.

12. Procédé selon l'une quelconque des revendications 1 à 10, **caractérisé en ce que** la seconde phase est une phase aqueuse et on utilise comme agent tensioactif au moins un agent tensioactif ionique et/ou non ionique, de préférence ayant une balance hydrophile/lipophile élevée.

13. Procédé selon l'une quelconque des revendications 1 à 12, **caractérisé en ce que** les nanocapsules obtenues ont un diamètre moyen compris entre 50 et 600 en ce que les nanocapsules obtenues ont un diamètre moyen compris entre 50 et 600 nm.

14. Procédé selon l'une quelconque des revendications 1 à 13, **caractérisé en ce que** le solvant ou le mélange de solvants de la première phase et le cas échéant le non-solvant ou le mélange de non-solvants de la seconde phase sont éliminés au moins pour partie par évaporation ou par une méthode de dessiccation appropriée ou par ultrafiltration tangentielle.

15. Procédé selon l'une quelconque des revendications 1 à 14, **caractérisé en ce que** qu'on obtient une poudre de nanocapsules après addition à la suspension colloïdale obtenue à l'étape (4) de substances stabilisantes telles que des sucres glucose et lactoses ; et mise en oeuvre d'une technique de dessiccation (lyophilisation, nébulisation).

## Claims

1. Process for the preparation of dispersible colloidal systems in the form of nanocapsules whose wall consists of a polymer obtained by the polycondensation of two monomers, α and β, and whose core consists of a substance B, **characterized in that**:
(1) a first liquid phase is prepared which consists of a solution of monomer α in a solvent or solvents mixture and contains at least one surfactant and the substance B in solution or suspension;
(2) a second liquid phase is prepared which consists of a non-solvent or non-solvent mixture for the monomer α and the substance B and contains the monomer β and at least one surfactant, the solvent or solvents mixture of the first phase being miscible in all proportions with the non-solvent or non-solvents mixture of the second phase, and the concentration of the monomer β being in at least a 5-fold excess, in terms of the number of moles, relative to the concentration of the monomer α;
(3) the first phase is added to the second, with moderate agitation, to give a colloidal suspension of nanocapsules, agitation being maintained until the monomers α and β have completely polymerized; and
(4) if desired, all or part of the solvent or solvents mixture and non-solvent or non-solvent mixture is removed to give a colloidal suspension having the desired concentration of nanocapsules.

2. Process according to claim 1, **characterized in that** the solvent or solvent mixture of the first phase is an organic solvent or organic solvents mixture and the non-solvent or non-solvents mixture of the second phase constitutes an aqueous phase.

3. Process according to claim 1 or 2, **characterized in that** the concentration of the monomer α in the solvent or solvents mixture of the first phase is between 0.01 and 20% by weight, preferably between 0.1 and 10% and particularly preferably between 0.2 and 5%.

4. Process according to any one of claims 1 to 3, **characterized in that** the concentration of the monomer β in the non-solvent or non-solvents mixture of the second phase is between 0.05 and 50% by weight, preferably between 0.5 and 40% and particularly preferably between 1 and 25%.

5. Process according to any one of claims 1 to 4, **characterized in that** the monomer α is selected from acid dichlorides and diisocyanates.

6. Process according to any one of claims 1 to 5, **characterized in that** the monomer β is a diamine or a glycol derivative.

7. Process according to any one of claims 1 to 6, **characterized in that** the substance B is a vegetable or mineral oil or any oily substance.

8. Process according to any one of claims 1 to 6, **characterized in that** the substance B is a biologically active substance.

9. Process according to any one of claims 1 to 6, **characterized in that** the substance B is an oil containing one or more biologically active substances in solution or suspension.

10. Process according to any one of claims 1 to 9, **characterized in that** the surfactant or surfactant mixture is present in each phase in an amount of 0.01 to 10% by weight, preferably of 0.1 to 1% by weight.

11. Process according to any one of claims 1 to 10, **characterized in that** the first phase is an organic phase and the surfactant used is at least one amphoteric and/or non-ionic surfactant, preferably with a low hydrophilic/lipophilic balance.

12. Process according to any one of claims 1 to 10, **characterized in that** the second phase is an aqueous phase and the surfactant used is at least one ionic and/or non-ionic surfactant, preferably with a high hydrophilic/lipophilic balance.

13. Process according to any one of claims 1 to 12, **characterized in that** the nanocapsules obtained have a mean diameter of between 50 and 600 nm.

14. Process according to any one of claims 1 to 13, **characterized in that** the solvent or solvents mixture of the first phase and, if appropriate, the non-solvent or non-solvents mixture of the second phase are at least partially removed by evaporation, by an appropriate desiccation method or by tangential ultrafiltration.

15. Process according to any one of claims 1 to 14, **characterized in that** a powder of nanocapsules is obtained after the addition of stabilizing substances, such as sugars, glucose and lactoses ; to the colloidal suspension obtained in step (4) and after the application of a desiccation technique (lyophilization, nebulization).

## Patentansprüche

1. Verfahren zur Herstellung von dispergierbaren kolloidalen Systemen in Form von Nanokapseln, deren Wand aus einem Polymer besteht, das durch Polykondensation von zwei Monomeren α und β erhalten wird, und deren Kern aus einer Substanz B besteht, **dadurch gekennzeichnet, daß**:
(1) eine erste Flüssigphase zubereitet wird, die aus einer Lösung des Monomers α in einem Lösungsmittel oder einem Gemisch von Lösungsmitteln besteht und die mindestens einen oberflächenaktiven Stoff sowie die Substanz B in Lösung oder in Suspension enthält,
(2) eine zweite Flüssigphase zubereitet wird, die aus einem Nicht-Lösungsmittel oder einem Gemisch aus Nicht-Lösungsmitteln des Monomers α und der Substanz B besteht, enthaltend das Monomer β und mindestens einen oberflächenaktiven Stoff, wobei das Lösungsmittel oder das Gemisch aus Lösungsmitteln der ersten Phase in jeglichem Verhältnis mit dem Nicht-Lösungsmittel oder dem Gemisch aus Nicht-Lösungsmitteln der zweiten Phase mischbar ist und die Konzentration des Monomers β in Bezug auf die Konzentration des Monomers α eine mindestens um das Fünffach größere Molzahl aufweist,
(3) bei mäßigem Rühren die erste Phase der zweiten Phase derart beigemengt wird, daß eine kolloidale Suspension von Nanokapseln erhalten wird, wobei das Rühren bis zur vollständigen Polymerisation der Monomere α und β aufrechterhalten wird,
(4) falls gewünscht, das Lösungsmittel oder das Gemisch aus Lösungsmitteln und das Nicht-Lösungsmittel oder das Gemisch aus Nicht-Lösungsmitteln derart vollständig oder teilweise so entfernt wird, daß eine kolloidale Suspension in Form von Nanokapseln in der gewünschten Konzentration erhalten wird.

2. Verfahren nach Anspruch 1, **dadurch gekennzeichnet, daß** das Lösungsmittel oder das Gemisch aus Lösungsmitteln der ersten Phase ein organisches Lösungsmittel oder ein Gemisch aus organischen Lösungsmitteln ist und das Nicht-Lösungsmittel oder das Gemisch aus Nicht-Lösungsmitteln der zweiten Phase eine wässerige Phase bilden.

3. Verfahren nach Anspruch 1 oder 2, **dadurch gekennzeichnet, daß** die Konzentration des Monomers α in dem Lösungsmittel oder dem Gemisch aus Lösungsmitteln der ersten Phase zwischen 0,01 und 20 Gew.-%, vorzugsweise zwischen 0,1 und 10 Gew.-% und am Bevorzugtesten zwischen 0,2 und 5 Gew.-% liegt.

4. Verfahren nach einem der Ansprüche 1 bis 3, **dadurch gekennzeichnet, daß** die Konzentration des Monomers β in dem Nicht-Lösungsmittel oder dem Gemisch aus Nicht-Lösungsmitteln der zweiten Phase zwischen 0,05 und 50 Gew.-%, vorzugsweise zwischen 0,5 und 40 Gew.-% und am bevorzugtesten zwischen 1 und 25 Gew.-% liegt.

5. Verfahren nach einem der Ansprüche 1 bis 4, **dadurch gekennzeichnet, daß** das Monomer α aus Säuredichloriden und Diisocyanaten ausgewählt ist.

6. Verfahren nach einem der Ansprüche 1 bis 5, **dadurch gekennzeichnet, daß** das Monomer β ein Diamin oder ein Glycolderivat ist.

7. Verfahren nach einem der Ansprüche 1 bis 6, **dadurch gekennzeichnet, daß** die Substanz B ein Pflanzenöl oder Mineral oder eine beliebige ölige Substanz ist.

8. Verfahren nach einem der Ansprüche 1 bis 6, **dadurch gekennzeichnet, daß** die Substanz B eine biologisch aktive Substanz ist.

9. Verfahren nach einem der Ansprüche 1 bis 6, **dadurch gekennzeichnet, daß** die Substanz B ein Öl ist, das eine oder mehrere Substanzen enthält, die in Lösung oder in Suspension biologisch aktiv ist (sind).

10. Verfahren nach einem der Ansprüche 1 bis 9, **dadurch gekennzeichnet, daß** der oberflächenaktive Stoff oder das Gemisch aus oberflächenaktiven Stoffen in jeder Phase im Verhältnis von 0,01 bis 10 Gew.-%, vorzugsweise 0,1 bis 1 Gew.-% vorhanden ist.

11. Verfahren nach einem der Ansprüche 1 bis 10, **dadurch gekennzeichnet, daß** die erste Phase eine organische Phase ist und als oberflächenaktiver Stoff mindestens ein amphoterer und/oder nicht-ionischer oberflächenaktiver Stoff verwendet wird, der vorzugsweise einen niedrigen HLB-Wert aufweist.

12. Verfahren nach einem der Ansprüche 1 bis 10, **dadurch gekennzeichnet, daß** die zweite Phase eine wässerige Phase ist und als oberflächenaktiver Stoff mindestens ein ionischer und/oder nicht ionischer oberflächenaktiver Stoff verwendet wird, der vorzugsweise einen hohen HLB-Wert aufweist.

13. Verfahren nach einem der Ansprüche 1 bis 12, **dadurch gekennzeichnet, daß** die erhaltenen Nanokapseln einen durchschnittlichen Durchmesser zwischen 50 und 600 nm aufweisen.

14. Verfahren nach einem der Ansprüche 1 bis 13, **dadurch gekennzeichnet, daß** das Lösungsmittel oder das Gemisch aus Lösungsmitteln der ersten Phase und gegebenenfalls das Nicht-Lösungsmittel oder das Gemisch aus Nicht-Lösungsmitteln der zweiten Phase mindestens teilweise durch Verdampfung oder durch ein geeignetes Trocknungsverfahren oder durch Tangential-Ultrafiltration entfernt werden.

15. Verfahren nach einem der Ansprüche 1 bis 14, **dadurch gekennzeichnet, daß** ein Pulver von Nanokapseln erhalten wird, nachdem zur in Schritt (4) erhaltenen kolloidalen Suspension stabilisierende Substanzen, wie Zucker, Glukose und Lactosen, hinzugefügt wurden; und Anwendung einer Trocknungstechnik (Gefriertrocknung, Zerstäubung).
